# EUROPEAN PATENT APPLICATION

(11) **EP 4 793 303 A1**
(43) Date of publication of application: **19.08.2026**
(21) Application number: 26158241.5
(22) Date of filing: 12.02.2026
(51) Int. Cl.: C08G 18/40, A61K 8/02, C08G 18/76, C08J 9/30

(54) **POLYURETHANE FOAM FOR COSMETIC COMPOSITION IMPREGNATION AND METHOD OF MANUFACTURING THE SAME**

(30) Priority: 14.02.2025 KR 20250019669
(71) Applicant: Kolmar Korea Co., Ltd., Sejong 30004 (KR)
(72) Inventor: PARK, Hee Ho, Seocho-gu, Seoul (KR); TAK, Gi Min, Seocho-gu, Seoul (KR); KIM, Yong Woo, Seocho-gu, Seoul (KR); LEE, Ji Hyun, Seocho-gu, Seoul (KR)
(74) Representative: Gulde & Partner

(57) **Abstract**

According to an embodiment of the present application, a polyurethane foam for cosmetic composition impregnation is provided. The polyurethane foam for cosmetic composition impregnation can be formed by mixing a polyether polyol and a polyester polyol in a weight ratio of 1:0.17 to 0.33 and foaming the mixture. According to embodiments of the present application, a polyurethane foam for cosmetic composition impregnation, which has improved long-term stability, can be easily filled and loaded with a cosmetic composition, and can easily discharge the cosmetic composition, can be provided.

## Description

### BACKGROUND

### 1. FIELD

The present application relates to a polyurethane foam for cosmetic composition impregnation and a method of manufacturing the same.

### 2. DISCUSSION OF THE BACKGROUND

In the modern cosmetics market, impregnation members such as sponges are widely used to load color cosmetics such as foundation. As the material for the impregnation member, various materials are used, and polyurethane foam is being increasingly used due to its porosity, elasticity, light weight, and the like.

Polyurethane is synthesized by chemically reacting polyols and diisocyanates. Here, the polyol for manufacturing polyurethane is classified into a polyether-based polyol and a polyester-based polyol. Polyurethane manufactured using a polyester-based polyol has the advantage of withstanding repeated pressure and friction when cosmetics are applied due to having excellent physical durability such as tensile strength, abrasion resistance, tear resistance, and the like. On the other hand, polyurethane manufactured using a polyether-based polyol has the advantage of maintaining its shape and function even in a highly moist environment due to having excellent moisture resistance. Therefore, there is a need to appropriately combine the advantages of polyurethane manufactured using a polyester-based polyol and the advantages of polyurethane manufactured using a polyether-based polyol and to manufacture them with physical properties optimized for use as impregnation members.

### SUMMARY

The present application is directed to providing a polyurethane foam for cosmetic composition impregnation and a method of manufacturing the same.

According to one aspect of the present application, there is provided a polyurethane foam for cosmetic composition impregnation. The polyurethane foam for cosmetic composition impregnation may be formed by mixing a polyether polyol and a polyester polyol in a weight ratio of 1:0.17 to 0.33 and foaming the mixture.

In addition, the polyurethane foam for cosmetic composition impregnation may have a pore number per inch of 70 to 80 ppi and a hardness of 70 to 85 based on the Asker durometer Type F.

Additionally, the polyurethane foam for cosmetic composition impregnation may have a tensile strength of 220 to 240 kPa and an elongation rate of 210 to 250%.

In addition, the polyurethane foam for cosmetic composition impregnation may be impregnated with a cosmetic composition having an oil-phase ingredient content of 25 wt% to 50 wt%.

Additionally, the polyurethane foam for cosmetic composition impregnation may be impregnated with a cosmetic composition having a viscosity of 7,000 cps or more and 45,000 cps or less.

According to another aspect of the present application, there is provided a method of manufacturing polyurethane foam for cosmetic composition impregnation. The method of manufacturing polyurethane foam for cosmetic composition impregnation includes mixing a composition for manufacturing polyurethane foam including a polyether polyol and a polyester polyol; and foaming the mixed polyurethane foam composition, wherein the polyether polyol and the polyester polyol are mixed in a weight ratio of 1:0.17 to 0.33 in the composition for manufacturing polyurethane foam.

In addition, the polyurethane foam for cosmetic composition impregnation may have a pore number per inch of 70 to 80 ppi and a hardness of 70 to 85 based on the Asker durometer Type F.

Additionally, the polyurethane foam for cosmetic composition impregnation may have a tensile strength of 220 to 240 kPa and an elongation rate of 210 to 250%.

In addition, the polyurethane foam for cosmetic composition impregnation may be impregnated with a cosmetic composition having an oil-phase ingredient content of 25 wt% to 50 wt%.

Additionally, the polyurethane foam for cosmetic composition impregnation may be impregnated with a cosmetic composition having a viscosity of 7,000 cps or more and 45,000 cps or less.

In addition, the composition for manufacturing polyurethane foam may include the polyether polyol, the polyester polyol, a foaming agent, a catalyst, and an additive.

Additionally, the mixing of a composition for manufacturing polyurethane foam may be performed at a temperature of 18 to 28 °C and a stirring speed of 4,000 to 5,000 rpm for 20 to 40 minutes.

In addition, the foaming may be performed at a discharge pressure of 35,000 to 45,000 Pa.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 shows the results of evaluating the stability of polyurethane foams according to manufacturing examples of the present application; and
FIGS. 2 and 3 show the results of evaluating the impregnation performance of polyurethane foams according to manufacturing examples of the present application.

### DETAILED DESCRIPTION

Structural or functional descriptions of embodiments disclosed in the present specification are merely illustrative for the purpose of describing the embodiments according to the technical idea of the present application. In addition, the embodiments according to the technical idea of the present application may be implemented in various other forms in addition to the embodiments disclosed in the present specification. Further, the technical idea of the present application should not be construed as limited to the embodiments described in the present specification.

Unless otherwise defined, all technical and scientific terms used in the present specification have the same meaning as commonly understood by those skilled in the art.

In terms of the physical properties described in the present specification, when a temperature at the time of measurement affects the physical properties, unless otherwise specified, the physical properties are those measured at room temperature and normal pressure.

In terms of the physical properties described in the present specification, unless otherwise specified, the physical properties may basically follow the SI unit system. For example, weight may be expressed in kilograms (kg), and length may be expressed in meters (m).

The term "room temperature" used in the present specification refers to a natural temperature in an unheated and uncooled state and may be, for example, any temperature ranging from 10 °C to 30 °C such as about 25 °C. Unless otherwise specified in the present specification, the unit of temperature is degrees Celsius (°C).

The term "normal pressure" used in the present specification refers to a natural pressure in a state where the pressure is not increased and decreased and is generally atmospheric pressure ranging from about 700 mmHg to 800 mmHg.

The phrase "a to b" used in the present specification refers to a range of a to b including a and b. For example, including a to b parts by weight is the same as including a range of a to b parts by weight.

Throughout the present specification, the values expressed in range format should be flexibly interpreted to include not only the numerical values explicitly stated as limits of such a range, but also all of the individual numerical values or subranges included in such a range, as if each numerical value and subrange are explicitly stated. For example, a range of "about 0.1% to about 5%" or "about 0.1% to 5%" should be interpreted to include not only about 0.1% to about 5%, but also individual values (e.g., 1%, 2%, 3%, and 4%) and subranges (e.g., 0.1% to 0.5%, 1.1% to 2.2%, and 3.3% to 4.4%) within the indicated range.

When a method for measuring or evaluating a specific dimension, size, formulation, parameter, shape, and other quantities and characteristics is not described in the present specification, those skilled in the art can easily recognize a conventionally known method for measuring or evaluating the same or can easily derive the measurement or evaluation method by referring to a known technology known prior to the filing date of the present specification.

The terminology used in the present specification is merely used for the purpose of describing embodiments, and is not intended to be limiting of the present application. The singular forms are intended to include the plural forms as well, unless the context clearly indicates otherwise. In addition, numbers (e.g., first, second, and etc.) used in the present specification are merely identifiers to distinguish one component from another.

In the present specification, when a portion "includes" an element, another element may be further included, rather than excluding the other element, unless otherwise described.

The term "or" used in the present application is intended to mean an inclusive "or" rather than an exclusive "or." In other words, unless otherwise specified or clear from context, "X uses A or B" is intended to mean one of the natural inclusive permutations. For the case in which X uses A; X uses B; or X uses both A and B, "X uses A or B" may be applied to any of the above cases. In addition, the term "and/or" used in the present specification should be understood to refer to and include all possible combinations of one or more of the listed relevant configurations.

According to one aspect of the present application, there is provided a polyurethane foam for cosmetic composition impregnation. Polyurethane foam is a material synthesized by chemically reacting a polyol and a diisocyanate, and is suitable as the material of a member for cosmetic composition impregnation due to having porosity, elasticity, light weight, and the like. The polyol is a compound with a plurality of hydroxyl groups (-OH), and the diisocyanate is a compound with two isocyanate groups (-NCO).

The polyurethane foam for cosmetic composition impregnation may be formed by foaming a composition for manufacturing polyurethane foam. Here, the composition for manufacturing polyurethane foam includes a polyether polyol and a polyester polyol. In other words, the polyurethane foam for cosmetic composition impregnation is formed by mixing a polyether polyol and a polyester polyol and foaming the mixture. The polyether polyol is a polyol with repeating ether bonds (-O-) and may include, for example, polypropylene glycol (PPG), polyethylene glycol (PEG), polyoxypropylene triol, polytetramethylene ether glycol (PTMEG), and the like. The polyester polyol is a polyol with repeating ester bonds (-COO-) and may include an adipic acid-based polyester polyol, a phthalic acid-based polyester polyol, a succinic acid-based polyester polyol, and the like.

The composition for manufacturing polyurethane foam includes a polyether polyol and a polyester polyol in a weight ratio of 1:0.17 to 0.33. In other words, the polyurethane foam for cosmetic composition impregnation is formed by mixing a polyether polyol and a polyester polyol in a weight ratio of 1:0.17 to 0.33 and foaming the mixture. In the present specification, the phrase "A and B are included/mixed and foamed in a weight ratio of X:Y" means that the "A : B weight ratio is X:Y." Within the above range, the cosmetic impregnation performance and long-term stability of the polyurethane foam can be improved. For example, the weight ratio of a polyether polyol and a polyester polyol may be 1:0.17 or more, 1:0.19 or more, 1:0.21 or more, or 1:0.23 or more. For example, the weight ratio of a polyether polyol and a polyester polyol may be 1:0.33 or less, 1:0.31 or less, 1:0.29 or less, or 1:0.27 or less.

In an embodiment, the polyurethane foam for cosmetic composition impregnation may have a pore number per inch of 70 to 80 ppi. The pore number per inch is a value obtained by measuring the number of pores present per inch in the polyurethane foam, and it can be understood that as the value is higher, the polyurethane foam has a structure having many small pores, and as the value is lower, the polyurethane foam has a structure having large pores formed at wide intervals. When the pore number per inch falls within the above range, the cosmetic impregnation performance and long-term stability of the polyurethane foam can be improved. For example, the pore number per inch of the polyurethane foam for cosmetic composition impregnation may be 70 ppi or more, 71 ppi or more, 72 ppi or more, 73 ppi or more, or 74 ppi or more. For example, the pore number per inch of the polyurethane foam for cosmetic composition impregnation may be 80 ppi or less, 79 ppi or less, 78 ppi or less, 77 ppi or less, or 76 ppi or less.

In an embodiment, the polyurethane foam for cosmetic composition impregnation may have a hardness of 70 to 85 based on the Asker durometer Type F. The Asker durometer is a hardness measuring system used to measure the hardness of soft materials such as rubber, foam, and gel, and the Asker durometer Type F is mainly used to measure the hardness of soft foam materials such as polyurethane foam and sponges. When the Asker hardness falls within the above range, the cosmetic impregnation performance and long-term stability of the polyurethane foam can be improved. For example, the hardness of the polyurethane foam for cosmetic composition impregnation may be 70 or more, 72 or more, 74 or more, 76 or more, or 78 or more. For example, the hardness of the polyurethane foam for cosmetic composition impregnation may be 85 or less, 84 or less, 83 or less, 82 or less, or 81 or less.

In an embodiment, the polyurethane foam for cosmetic composition impregnation may have a tensile strength of 200 to 300 kPa. The tensile strength is a value indicating that how much force a material can withstand before it breaks or fractures. When the tensile strength falls within the above range, the cosmetic impregnation performance and long-term stability of the polyurethane foam can be improved. For example, the tensile strength of the polyurethane foam for cosmetic composition impregnation may be 200 kPa or more, 210 kPa or more, 220 kPa or more, 230 kPa or more, or 240 kPa or more. For example, the tensile strength of the polyurethane foam for cosmetic composition impregnation may be 300 kPa or less, 290 kPa or less, 280 kPa or less, 270 kPa or less, 260 kPa or less, 250 kPa or less, or 240 kPa or less. For example, the tensile strength of the polyurethane foam for cosmetic composition impregnation may be 220 to 240 kPa.

In an embodiment, the polyurethane foam for cosmetic composition impregnation may have an elongation rate of 200 to 250%. The elongation rate is a value indicating that how much a material can stretch before it breaks and is expressed as a percentage. When the elongation rate falls within the above range, the cosmetic impregnation performance and long-term stability of the polyurethane foam can be improved. For example, the elongation rate of the polyurethane foam for cosmetic composition impregnation may be 200% or more, 210% or more, or 220% or more. For example, the elongation rate of the polyurethane foam for cosmetic composition impregnation may be 250% or less, 240% or less, or 230% or less. For example, the elongation rate of the polyurethane foam for cosmetic composition impregnation may be 210 to 250%.

In an embodiment, the polyurethane foam for cosmetic composition impregnation may be impregnated with a cosmetic composition having an oil-phase ingredient content of 25 wt% to 50 wt%. Here, the oil-phase ingredient may refer to a fat-soluble ingredient such as an oil (e.g., a vegetable oil, an animal oil, a mineral oil, a synthetic oil, etc.), a wax, a higher fatty acid, a higher alcohol, an ester-based oil, a silicone-based oil, an organic UV blocker, or the like. When the polyurethane foam is impregnated with a cosmetic composition having an oil-phase ingredient content of 25 wt% to 50 wt%, excellent impregnation performance can be exhibited. For example, the oil-phase ingredient content of a cosmetic composition may be 25 wt% or more, 30 wt% or more, 35 wt% or more, 40 wt% or more, or 45 wt% or more. For example, the oil-phase ingredient content of a cosmetic composition may be 50 wt% or less, 45 wt% or less, 40 wt% or less, 35 wt% or less, or 30 wt% or less.

In an embodiment, the polyurethane foam for cosmetic composition impregnation may be impregnated with a cosmetic composition having a viscosity of 5,000 cps or more and 45,000 cps or less. For example, the viscosity of the impregnated cosmetic composition may be equal to or greater than 5,000 cps, 7,000 cps, 10,000 cps, 15,000 cps, 20,000 cps, 25,000 cps, or 30,000 cps, or equal to or less than 45,000 cps, 40,000 cps, 37,000 cps, 35,000 cps, 30,000 cps, 25,000 cps, or 20,000 cps. For example, the viscosity of the cosmetic composition may be 7,000 cps or more and 45,000 cps or less. For example, the viscosity of the cosmetic composition may be 7,000 cps or more and 40,000 cps or less. For example, the viscosity of the cosmetic composition may be 10,000 cps or more and less than 40,000 cps. The viscosity is measured with a Brookfield DVplus Viscometer, Spindle No.4, at 6 rpm and 25°C.

In an embodiment, the cosmetic composition may include an additive typically used in the art within the range that does not impair the effects of the present application. For example, the cosmetic composition may further include a thickener, a pH adjusting agent, a surfactant, a stabilizer, a preservative, an antiseptic, a bactericide, a moisturizer, a sunscreen, a skin conditioning agent, a whitening agent, a colorant, an organic pigment, an inorganic pigment, a fragrance, a vitamin, a natural pigment, a pearl, other functional ingredients, and the like. Here, the functional ingredient may include, for example, a wrinkle improving ingredient, a whitening ingredient, an antioxidant ingredient, an anti-aging ingredient, a plumping ingredient, an exfoliating ingredient, a scrubbing ingredient, a moisturizing ingredient, a skin barrier strengthening ingredient, a skin elasticity strengthening ingredient, and the like. The blending amount of the ingredients may be easily selected by those skilled in the art within the range that does not impair the purpose and effects of the present application.

In an embodiment, the cosmetic composition may be provided in a formulation typically prepared in the art. For example, the cosmetic composition may be formulated into an oil dispersion formulation in which a powder complex is dispersed in an oil phase, an aqueous dispersion formulation in which a powder complex is dispersed in an aqueous phase, an emulsion formulation of oil-in-water (O/W), water-in-oil (W/O), water-in-oil-in-water (W/O/W), or oil-in-water-in-oil (O/W/O) type, a solubilized formulation, or the like. However, the present invention is not limited thereto, and the cosmetic composition may also be provided in a formulation such as a solution, a suspension, an emulsion, or the like.

In an embodiment, the cosmetic composition may be prepared in the form of various products. For example, it may be prepared into a sun care product such as a sunscreen, sun milk, sun lotion, or sun cushion. In addition, it may be prepared into a soap, detergent, or cleansing cream or into a color cosmetic such as a lipstick, makeup base, foundation, compact, concealer, or skin cover. Also, it may be prepared into a hair cleansing product such as a shampoo, rinse, hair conditioner, or hair gel.

In an embodiment, the composition may be a composition for an external preparation for skin. For example, the composition for an external preparation for skin may have a formulation such as a solution or the like.

In an embodiment, the composition for an external preparation for skin may include an ingredient used in an external preparation for skin of typical cosmetics or drugs, for example, an aqueous-phase ingredient, an oil-phase ingredient, a powdered ingredient, an alcohol, a moisturizer, a thickener, a sunscreen, a whitening agent, an antiseptic, an antioxidant, a surfactant, a fragrance, a coloring agent, a fatty substance, a solubilizer, a thickening agent, a gelling agent, a softener, a foaming agent, an air freshener, a metal ion sequestering agent, a chelating agent, a vitamin, various skin nutrients, or a combination thereof, which may be appropriately blended as necessary. The blending amount of the ingredients may be easily selected by those skilled in the art within the range that does not impair the purpose and effects of the present application.

According to another aspect of the present application, there is provided a method of manufacturing polyurethane foam for cosmetic composition impregnation. The method of manufacturing polyurethane foam for cosmetic composition impregnation includes mixing a composition for manufacturing polyurethane foam; and foaming the mixed polyurethane foam composition. The detailed description of a polyurethane foam for cosmetic composition impregnation manufactured by the above method is as described above in the present specification.

The composition for manufacturing polyurethane foam includes a polyether polyol and a polyester polyol. The detailed descriptions of the polyether polyol, the polyester polyol, and the mixing ratio thereof are as described above in the present specification.

In an embodiment, the composition for manufacturing polyurethane foam may include the polyether polyol, the polyester polyol, a foaming agent, a catalyst, and an additive. The foaming agent may form air bubbles in polyurethane foam to form a porous structure and may include, for example, water, hydrochlorofluorocarbon-141b (HCFC-141b), hydrofluorocarbon-245fa (HFC-245fa), and the like. The catalyst may control the curing time, mechanical properties, and final structure of polyurethane foam and may include, for example, dimethylethanolamine (DMEA), dibutyltin dilaurate (DBTDL), triethylenediamine (TEDA, DABCO), and the like. The additive may improve the physical and chemical properties of polyurethane foam or may impart a specific function to polyurethane foam and may include, for example, a silicone surfactant, tris(1-chloro-2-propyl) phosphate (TCPP), calcium carbonate, and the like. When the type and content of foaming agent, catalyst, and additive are appropriately adjusted, the physical properties of polyurethane foam can be optimized.

In an embodiment, the composition for manufacturing polyurethane foam may further include a reactant. The reactant may be, for example, a material with two or more isocyanate groups. For example, the reactant may be a diisocyanate. For example, the reactant may include toluene diisocyanate (TDI), diphenylmethane diisocyanate (MDI), naphthalene diisocyanate (NDI), hexamethylene diisocyanate (HDI), and the like.

In an embodiment, the mixing of a composition for manufacturing polyurethane foam may be performed at a temperature of 18 to 28 °C and a stirring speed of 4,000 to 5,000 rpm for 20 to 40 minutes. Under the above conditions, the physical properties of the polyurethane foam according to an embodiment of the present application can be exhibited.

In an embodiment, in the foaming, the polyether polyol and the polyester polyol may react with the diisocyanate to form polyurethane foam.

In an embodiment, the foaming may be performed at a discharge pressure of 35,000 to 45,000 Pa. Under the above condition, the physical properties of the polyurethane foam according to an embodiment of the present application can be exhibited.

Hereinafter, the present invention will be described with reference to manufacturing examples and experimental examples, and the scope of the present invention is not limited to the following examples.

### Manufacturing Examples

### (1) Manufacture of Manufacturing Examples 1 to 3

Polyol compositions according to the contents shown in Table 1 were each mixed with methylene diphenylmethane diisocyanate (MDI) in a weight ratio of 1:1 at a temperature of 18 to 28 °C and a stirring speed of 4,500 rpm for 30 minutes, and then the resulting mixture was foamed at a discharge pressure of 40,000 Pa to manufacture polyurethane foams of Manufacturing Examples 1 to 3. The content units in Table 1 are wt%.

**[Table 1]**

| Classification | | Manufacturing Example 1 | Manufacturing Example 2 | Manufacturing Example 3 |
|---|---|---|---|---|
| Polyol composition | Polyether polyol | 78.1 | 68.1 | - |
| | Polyester polyol | 19.2 | 29.2 | 97.3 |
| | Others | 2.7 | 2.7 | 2.7 |
| Total | | 100 | 100 | 100 |

### (2) Manufacture of Manufacturing Examples 4 and 5

The physical properties of the polyurethane foam manufactured according to Manufacturing Example 1 are shown in Table 2. Also, polyurethane foams of Manufacturing Examples 4 and 5 were manufactured by varying the physical properties of polyurethane foam as shown in Table 2 while changing the conditions for the process of manufacturing polyurethane foam.

The pore number per inch (ppi) of Manufacturing Examples 1, 4, and 5 was measured in accordance with ASTM WI-QA-14, the Asker hardness was measured in accordance with JSI K6301A, the elongation rate was measured in accordance with ASTM D638, and the tensile strength was measured in accordance with ISO 1798.

**[Table 2]**

| | Manufacturing Example 1 | Manufacturing Example 4 | Manufacturing Example 5 |
|---|---|---|---|
| Pore number per inch (ppi) | 75 | 45 | 100 |
| Asker (type F) hardness | 80 | 50 | 90 |
| Elongation rate (%) | 232 | 263 | 208 |
| Tensile strength (kPa) | 237 | 245 | 212 |

### Experimental Examples

### (1) Experimental Example 1: Evaluation of stability of polyurethane foam

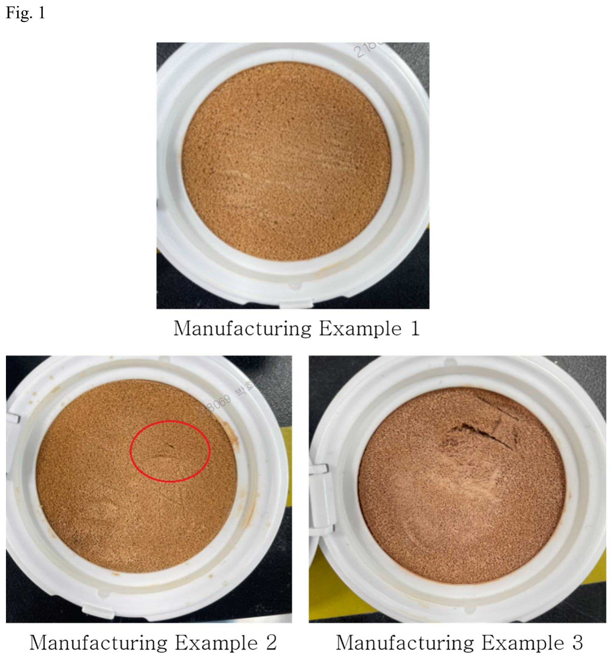

Each polyurethane foam of Manufacturing Examples 1 to 3 was input into an incubator set at a temperature of 70 °C and a humidity of 90%, whether hydrolysis occurred and whether elastic recovery was maintained were visually observed over time, and results are shown in Table 3. Here, whether elastic recovery was maintained was evaluated by pressing each polyurethane foam with a sharp object for 1 second and then determining whether tearing occurred. Also, photographs taken after each polyurethane foam of Manufacturing Examples 1 to 3 was pressed with a sharp object for 1 second after 3 months of being input in the incubator are shown in FIG. 1.

**[Table 3]**

| | After 1 day | After 2 weeks | After 1 month | After 2 months | After 3 months |
|---|---|---|---|---|---|
| Manufacturing Example 1 | - | - | - | - | Elastic recovery was maintained |
| Manufacturing Example 2 | - | - | - | - | Elastic recovery was degraded |
| Manufacturing Example 3 | - | Hydrolyzed | Hydrolyzed | Hydrolyzed | Hydrolyzed |

As experimental results, in the case of the polyurethane foam of Manufacturing Example 1, it can be confirmed that hydrolysis did not occur and elastic recovery was maintained even after 3 months.

On the other hand, in the case of Manufacturing Example 2, it was confirmed that elastic recovery was degraded after 3 months. In the case of Manufacturing Example 3, it was confirmed that hydrolysis occurred after 2 weeks.

### (2) Experimental Example 2: Evaluation of cosmetic composition impregnation performance of polyurethane foam

The cosmetic impregnation performance of Manufacturing Examples 1, 4, and 5 was evaluated in terms of fillability, loadability, and dischargeability, and results are shown in Table 4. A case in which fillability, loadability, or dischargeability was excellent was evaluated as O, and a case in which each item was poor was evaluated as X.

Specifically, in terms of fillability, 10 g of a cosmetic composition having an oil-phase ingredient content of 40% and a viscosity of 20,000 cps was applied onto the polyurethane foam, and 1 day later, whether the cosmetic composition was deposited inside the polyurethane foam was confirmed. In this case, a case in which the cosmetic composition was completely deposited was evaluated as excellent fillability, and a case in which the cosmetic composition remained undeposited was evaluated as poor fillability.

In addition, in terms of loadability, 10 g of a cosmetic composition having an oil-phase ingredient content of 40% and a viscosity of 20,000 cps was applied onto the polyurethane foam, and the change in weight of the polyurethane foam before and after impregnation was measured to determine the total amount (g) of the cosmetic composition loaded. A case in which the loading amount was 9 g or more was evaluated as excellent loadability, and a case in which the loading amount was less than 9 g was evaluated as poor loadability.

Additionally, in terms of dischargeability, the polyurethane foam impregnated with the cosmetic composition was pressed with a pressure of 100 N/m² using a cosmetic puff to apply the cosmetic composition onto the cosmetic puff, and the change in weight of the cosmetic puff before and after application was measured to determine the total amount (g) of the cosmetic composition discharged. A case in which the discharge amount was 0.2 to 0.4 g was evaluated as excellent dischargeability, a case in which the discharge amount was less than 0.2 g was evaluated as poor dischargeability due to an insufficient discharge amount, and a case in which the discharge amount exceeds 0.4 g was evaluated as poor dischargeability because its use was difficult due to an excessively large discharge amount and the cosmetic flowed out of the polyurethane foam even without an external force.

**[Table 4]**

| Classification | Fillability | Loadability | Dischargeability |
|---|---|---|---|
| Manufacturing Example 1 | O (completely deposited after 1 day) | O (9.8 g) | O (0.25 g) |
| Manufacturing Example 4 | O (completely deposited after 1 day) | O (9.8 g) | X (0.45 g) |
| Manufacturing Example 5 | X (cosmetic composition remained on the surface after 1 day) | X (loading was not possible) | X (0.10 g) |

As experimental results, in the case of Manufacturing Example 1, it can be confirmed that fillability, loadability, and dischargeability were all excellent, and thus it was suitable for cosmetic composition impregnation.

On the other hand, in the case of Manufacturing Example 4, it can be confirmed that its use was difficult due to an excessively large cosmetic discharge amount, and the cosmetic flowed out of the polyurethane foam even without an external force, thereby exhibiting poor dischargeability. In the case of Manufacturing Example 5, it can be confirmed that fillability, loadability, and dischargeability were overall poor.

### (3) Experimental Example 3: Evaluation of impregnation performance according to oil-phase ingredient content and viscosity of cosmetic composition

The cosmetic impregnation performance of the polyurethane foam of Manufacturing Example 1 was evaluated in terms of fillability, loadability, and dischargeability by varying the oil-phase ingredient content and viscosity of an impregnated cosmetic composition as shown in Table 5, and results are shown in Table 5. Fillability, loadability, and dischargeability were evaluated by the same method as in Experimental Example 2.

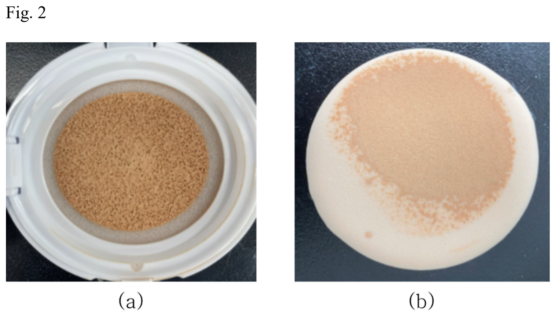

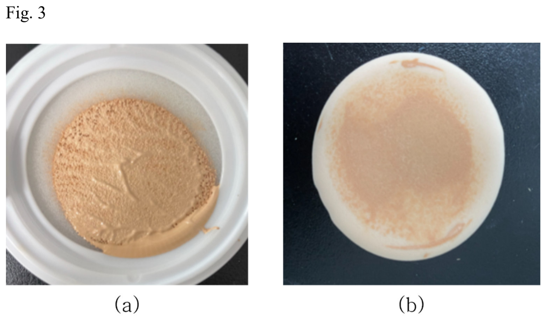

A photograph of the polyurethane foam of Manufacturing Example 1 filled with a cosmetic composition having an oil-phase ingredient content of 40 wt% and a viscosity of 20,000 cps is shown in FIG. 2A, and a photograph of a cosmetic puff to which the cosmetic composition was applied by pressing the filled polyurethane foam is shown in FIG. 2B. Also, a photograph of the polyurethane foam of Manufacturing Example 1 filled with a cosmetic composition having an oil-phase ingredient content of 20 wt% and a viscosity of 50,000 cps is shown in FIG. 3A, and a photograph of a cosmetic puff to which the cosmetic composition was applied by pressing the filled polyurethane foam is shown in FIG. 3B.

**[Table 5]**

| Classification | | Impregnation performance | | |
|---|---|---|---|---|
| Oil-phase ingredient content (wt%) | Viscosity (cps) | Fillability | Loadability | Dischargeability |
| 20 | 50,000 | X (cosmetic composition remained on the surface after 1 day) | X (8.0 g) | X (0.12 g) |
| 25 | 35,000 | O (completely deposited after 1 day) | O (9.8 g) | O (0.22 g) |
| 40 | 20,000 | O (completely deposited after 1 day) | O (9.8 g) | O (0.25 g) |
| 50 | 10,000 | O (completely deposited after 1 day) | O (9.8 g) | O (0.25 g) |
| 60 | 3,000 | O (completely deposited after 1 day) | O (9.9 g) | X (0.48 g) |

As experimental results, in the case of the polyurethane foam of Manufacturing Example 1, it can be confirmed that fillability, loadability, and dischargeability with respect to the cosmetic compositions having an oil-phase ingredient content of 25 to 50 wt% and a viscosity of 5,000 cps or more and 45,000 or less were all excellent, and thus it was suitable for cosmetic composition impregnation.

According to embodiments of the present application, a polyurethane foam for cosmetic composition impregnation, which has improved long-term stability, can be easily filled and loaded with a cosmetic composition, and can easily discharge the cosmetic composition, can be provided.

In addition, according to embodiments of the present application, a polyurethane foam, which is impregnated with a cosmetic composition having an oil-phase ingredient content of 25 wt% to 50 wt% and/or a viscosity of 5,000 cps or more and 45,000 cps or less with high long-term stability and impregnation performance, can be provided.

## Claims

1. A polyurethane foam for cosmetic composition impregnation, which is formed by mixing a polyether polyol and a polyester polyol in a weight ratio of 1:0.17 to 0.33 and foaming the mixture.

2. The polyurethane foam of claim 1, which has a pore number per inch of 70 to 80 ppi and a hardness of 70 to 85 based on the Asker durometer Type F and measured in accordance with ASTM WI-QA-14.

3. The polyurethane foam of claim 1 or 2, which has a tensile strength of 220 to 240 kPa measured in accordance with ISO 1798 and an elongation rate of 210 to 250% measured in accordance with ASTM D638.

4. The polyurethane foam of any one of the preceding claims, which is impregnated with a cosmetic composition having an oil-phase ingredient content of 25 wt% to 50 wt%.

5. The polyurethane foam of any one of the preceding claims, which is impregnated with a cosmetic composition having a viscosity of 7,000 cps or more and 45,000 cps or less.

6. A method of manufacturing polyurethane foam for cosmetic composition impregnation, comprising:
mixing a composition for manufacturing polyurethane foam including a polyether polyol and a polyester polyol; and
foaming the mixed polyurethane foam composition,
wherein the polyether polyol and the polyester polyol are mixed in a weight ratio of 1:0.17 to 0.33 in the composition for manufacturing polyurethane foam.

7. The method of claim 6, wherein the composition for manufacturing polyurethane foam includes the polyether polyol, the polyester polyol, a foaming agent, a catalyst, and an additive.

8. The method of claim 6 or 7, wherein the mixing of a composition for manufacturing polyurethane foam is performed at a temperature of 18 to 28 °C and a stirring speed of 4,000 to 5,000 rpm for 20 to 40 minutes.

9. The method of any one of claims 6 to 8, wherein the foaming is performed at a discharge pressure of 35,000 to 45,000 Pa.
